# EUROPEAN PATENT SPECIFICATION

(11) **EP 2 185 683 B1**
(45) Date of publication and mention of the grant of the patent: **18.04.2012**
(21) Application number: 08792550.9
(22) Date of filing: 13.08.2008
(51) Int. Cl.: C12P 13/08

(54) **METHOD FOR PRODUCING L-THREONINE USING BACTERIUM OF ENTEROBACTERIACEAE FAMILY, HAVING ATTENUATED EXPRESSION OF THE YAHN GENE**
VERFAHREN ZUR HERSTELLUNG VON L-THREONIN UNTER VERWENDUNG EINES BAKTERIUMS DER FAMILIE ENTEROBACTERIACEAE MIT VERMINDERTER EXPRESSION DES YAHN-GENS
PROCÉDÉ DE PRODUCTION DE L-THREONINE AU MOYEN D'UNE BACTÉRIE DE LA FAMILLE DES ENTEROBACTERIACEAE, AYANT UNE EXPRESSION ATTÉNUÉE DU GÈNE YAHN

(30) Priority: 14.08.2007 RU 2007131007; 16.05.2008 US 53707 P
(43) Date of publication of application: 19.05.2010
(73) Proprietor: Ajinomoto Co., Inc., Tokyo 104-8315 (JP)
(72) Inventor: ALTMAN, Irina, Borisovna, Moscow 119435 (RU); PTITSYN, Leonid, Romanovich, Moscow 115404 (RU)
(74) Representative: HOFFMANN EITLE
(86) International application number: PCT/JP2008/064770
(87) International publication number: WO 2009/022754

(56) References cited:
- EP-A- 1 016 710
- WO-A-2006/057341
- ALESHIN V V ET AL: "A new family of amino-acid-efflux proteins" TRENDS IN BIOCHEMICAL SCIENCES, ELSEVIER, HAYWARDS, GB, vol. 24, no. 4, 1 April 1999 (1999-04-01), pages 133-135, XP004214249 ISSN: 0968-0004 cited in the application
- DATABASE UniProt [Online] 1 February 1997 (1997-02-01), "RecName: Full=Uncharacterized membrane protein yahN;" XP002136827 retrieved from EBI accession no. UNIPROT:P75693 Database accession no. P75693

### SEQUENCE LISTING

<110> Ajinomoto Co., Inc.
<120> METHOD FOR PRODUCING L-AMINO ACID USING BACTERIUM OF ENTEROBACTERIACEAE FAMILY, HAVING ATTENUATED EXPRESSION OF THE yahN GENE
<130> C881-C8169
<150> RU2007131007
   <151> 2007-08-14
<150> US61/053707
   <151> 2008-05-16
<160> 14
<170> PatentIn version 3.1
<210> 1
<211> 672
   <212> DNA
   <213> Escherichia Coli
<220>
   <221> CDS
   <222> (1).. (672)
<400> 1
   atg atg cag tta gtt cac tta ttt atg gat gaa atc act atg gat cct      48
   Met Met Gln Leu Val His Leu Phe Met Asp Glu Ile Thr Met Asp Pro
   1               5                   10                  15
   ttg cat gcc gtt tac ctg acc gta gga ctg ttc gtg att act ttt ttt      96
   Leu His Ala Val Tyr Leu Thr Val Gly Leu Phe Val Ile Thr Phe Phe
               20                  25                  30
   aat ccg gga gcc aat ctc ttt gtg gta gta caa acc agc ctg gct tcc      144
   Asn Pro Gly Ala Asn Leu Phe Val Val Val Gln Thr Ser Leu Ala Ser
           35                  40                  45
   ggt cga cgc gca ggg gtg ctg acc ggg ctg ggc gtg gcg ctg ggc gat      192
   Gly Arg Arg Ala Gly Val Leu Thr Gly Leu Gly Val Ala Leu Gly Asp
       50                  55                  60
   gca ttt tat tcc ggg ttg ggt ttg ttt ggt ctt gca acg cta att acg      240
   Ala Phe Tyr Ser Gly Leu Gly Leu Phe Gly Leu Ala Thr Leu Ile Thr
   65                  70                  75                  80
   cag tgt gag gag att ttt tcg ctt atc aga atc gtc ggc ggc gct tat      288
   Gln Cys Glu Glu Ile Phe Ser Leu Ile Arg Ile Val Gly Gly Ala Tyr
                   85                  90                  95
   ctc tta tgg ttt gcg tgg tgc agc atg cgc cgc cag tca aca ccg caa     336
   Leu Leu Trp Phe Ala Trp Cys Ser Met Arg Arg Gln Ser Thr Pro Gln
               100                 105                 110
   atg agc aca cta caa caa ccg att agc gcc ccc tgg tat gtc ttt ttt     384
   Met Ser Thr Leu Gln Gln Pro Ile Ser Ala Pro Trp Tyr Val Phe Phe
           115                 120                 125
   cgc cgc gga tta att acc gat ctc tct aac ccg caa acc gtt tta ttt     432
   Arg Arg Gly Leu Ile Thr Asp Leu Ser Asn Pro Gln Thr Val Leu Phe
       130                 135                 140
   ttt atc agt att ttc tca gta aca tta aat gcc gaa aca cca aca tgg     480
   Phe Ile Ser Ile Phe Ser Val Thr Leu Asn Ala Glu Thr Pro Thr Trp
   145                 150                 155                 160
   gca cgt tta atg gcc tgg gcg ggg att gtg ctc gca tca att atc tgg     528
   Ala Arg Leu Met Ala Trp Ala Gly Ile Val Leu Ala Ser Ile Ile Trp
                   165                 170                 175
   cga gtt ttt ctt agt cag gcg ttt tct ttg ccc gct gtg cgt cgt gct     576
   Arg Val Phe Leu Ser Gln Ala Phe Ser Leu Pro Ala Val Arg Arg Ala
               180                 185                 190
   tat ggg cgt atg caa cgc gtt gcc agt cgg gtt att ggt gca att att     624
   Tyr Gly Arg Met Gln Arg Val Ala Ser Arg Val Ile Gly Ala Ile Ile
           195                 200                 205
   ggt gta ttc gcg cta cgc ctg att tac gaa ggg gtg acg cag cgg tga     672
   Gly Val Phe Ala Leu Arg Leu Ile Tyr Glu Gly Val Thr Gln Arg
       210                 215                 220
<210> 2
<211> 223
   <212> PRT
<213> Escherichia coli
<400> 2
   Met Met Gln Leu Val His Leu Phe Met Asp Glu Ile Thr Met Asp Pro
   1               5                   10                  15
   Leu His Ala Val Tyr Leu Thr Val Gly Leu Phe Val Ile Thr Phe Phe
               20                  25                  30
   Asn Pro Gly Ala Asn Leu Phe Val Val Val Gln Thr Ser Leu Ala Ser
           35                  40                  45
   Gly Arg Arg Ala Gly Val Leu Thr Gly Leu Gly Val Ala Leu Gly Asp
       50                  55                  60
   Ala Phe Tyr Ser Gly Leu Gly Leu Phe Gly Leu Ala Thr Leu Ile Thr
   65                  70                  75                  80
   Gln Cys Glu Glu Ile Phe Ser Leu Ile Arg Ile Val Gly Gly Ala Tyr
                    85                  90                  95
   Leu Leu Trp Phe Ala Trp Cys Ser Met Arg Arg Gln Ser Thr Pro Gln
               100                 105                 110
   Met Ser Thr Leu Gln Gln Pro Ile Ser Ala Pro Trp Tyr Val Phe Phe
           115                 120                 125
   Arg Arg Gly Leu Ile Thr Asp Leu Ser Asn Pro Gln Thr Val Leu Phe
       130                 135                 140
   Phe Ile Ser Ile Phe Ser Val Thr Leu Asn Ala Glu Thr Pro Thr Trp
   145                 150                 155                 160
   Ala Arg Leu Met Ala Trp Ala Gly Ile Val Leu Ala Ser Ile Ile Trp
                   165                 170                 175
   Arg Val Phe Leu Ser Gln Ala Phe Ser Leu Pro Ala Val Arg Arg Ala
               180                 185                 190
   Tyr Gly Arg Met Gln Arg Val Ala Ser Arg Val Ile Gly Ala Ile Ile
           195                 200                 205
   Gly Val Phe Ala Leu Arg Leu Ile Tyr Glu Gly Val Thr Gln Arg
       210                 215                 220
<210> 3
<211> 64
   <212> DNA
   <213> artificial sequence
<220>
   <223> primer
<400> 3
   gcaaatcagg ttgattacgt agagagtaaa ttatctgctc acgttgtgtc tcaaaatctc    60
   tgat                                                                 64
<210> 4
   <211> 68
   <212> DNA
   <213> artificial sequence
<220>
   <223> primer
<400> 4
   ggaaataaga cactttgtga tctctctcgc acccgattat tccagaaaaa ctcatcgagc    60
   atcaaaag                                                             68
<210> 5
<211> 24
   <212> DNA
   <213> artificial sequence
<220>
   <223> primer
<400> 5
   tgaaaaaagc ccacacaggg gaga 24
<210> 6
<211> 24
   <212> DNA
   <213> artificial sequence
<220>
   <223> primer
<400> 6
   ttatcacact atcacccacc caca 24
<210> 7
<211> 70
   <212> DNA
   <213> artificial sequence
<220>
   <223> primer
<400> 7
   gatgaaagcg ttatccaaac tgaaagcgga agaggccgac gcactttgcg ccgaataaat 60
   acctgtgacg 70
<210> 8
<211> 69
   <212> DNA
   <213> artificial sequence
<220>
   <223> primer
<400> 8
   ttaatcccag ctcagaataa ctttcccgga ctttacgccc cgccctgcca ctcatcgcag 60
   tactgttgt 69
<210> 9
<211> 18
   <212> DNA
   <213> artificial sequence
<220>
   <223> primer
<400> 9
   cggtcatgct tggtgatg 18
<210> 10
<211> 21
   <212> DNA
   <213> artificial sequence
<220>
   <223> primer
<400> 10
   ttaatcccag ctcagaataa c 21
<210> 11
   <211> 888
   <212> DNA
   <213> Escherichia coli
<220>
   <221> CDS
   <222> (1).. (885)
<400> 11
   atg cct ggt tca tta cgt aaa atg ccg gtc tgg tta cca ata gtc ata        48
   Met Pro Gly Ser Leu Arg Lys Met Pro Val Trp Leu Pro Ile Val Ile
   1               5                   10                  15
   ttg ctc gtt gcc atg gcg tct att cag ggt gga gcc tcg tta gct aag        96
   Leu Leu Val Ala Met Ala Ser Ile Gln Gly Gly Ala Ser Leu Ala Lys
               20                  25                  30
   tca ctt ttt cct ctg gtg ggc gca ccg ggt gtc act gcg ctg cgt ctg       144
   Ser Leu Phe Pro Leu Val Gly Ala Pro Gly Val Thr Ala Leu Arg Leu
           35                  40                  45
   gca tta gga acg ctg atc ctc atc gcg ttc ttt aag cca tgg cga ctg       192
   Ala Leu Gly Thr Leu Ile Leu Ile Ala Phe Phe Lys Pro Trp Arg Leu
       50                  55                  60
   cgc ttt gcc aaa gag caa cgg tta ccg ctg ttg ttt tac ggc gtt tcg       240
   Arg Phe Ala Lys Glu Gln Arg Leu Pro Leu Leu Phe Tyr Gly Val Ser
   65                  70                  75                  80
   ctg ggt ggg atg aat tat ctt ttt tat ctt tct att cag aca gta ccg       288
   Leu Gly Gly Met Asn Tyr Leu Phe Tyr Leu Ser Ile Gln Thr Val Pro
                   85                  90                  95
   ctg ggt att gcg gtg gcg ctg gag ttc acc gga cca ctg gcg gtg gcg       336
   Leu Gly Ile Ala Val Ala Leu Glu Phe Thr Gly Pro Leu Ala Val Ala
               100                 105                 110
   ctg ttc tct tct cgt cgc ccg gta gat ttc gtc tgg gtt gtg ctg gcg       384
   Leu Phe Ser Ser Arg Arg Pro Val Asp Phe Val Trp Val Val Leu Ala
           115                 120                 125
   gtt ctt ggt ctg tgg ttc ctg cta ccg ctg ggg caa gac gtt tcc cat       432
   Val Leu Gly Leu Trp Phe Leu Leu Pro Leu Gly Gln Asp Val Ser His
       130                 135                 140
   gtc gat tta acc ggc tgt gcg ctg gca ctg ggg gcc ggg gct tgt tgg       480
   Val Asp Leu Thr Gly Cys Ala Leu Ala Leu Gly Ala Gly Ala Cys Trp
   145                 150                 155                 160
   gct att tac att tta agt ggg caa cgc gca gga gcg gaa cat ggc cct       528
   Ala Ile Tyr Ile Leu Ser Gly Gln Arg Ala Gly Ala Glu His Gly Pro
                   165                 170                 175
   gcg acg gtg gca att ggt tcg ttg att gca gcg tta att ttc gtg cca       576
   Ala Thr Val Ala Ile Gly Ser Leu Ile Ala Ala Leu Ile Phe Val Pro
               180                 185                 190
   att gga gcg ctt cag gct ggt gaa gca ctc tgg cac tgg tcg gtt att       624
   Ile Gly Ala Leu Gln Ala Gly Glu Ala Leu Trp His Trp Ser Val Ile
           195                 200                 205
   cca ttg ggt ctg gct gtc gct att ctc tcg acc gct ctg cct tat tcg       672
   Pro Leu Gly Leu Ala Val Ala Ile Leu Ser Thr Ala Leu Pro Tyr Ser
       210                 215                 220
   ctg gaa atg att gcc ctc acc cgt ttg cca aca cgg aca ttt ggt acg       720
   Leu Glu Met Ile Ala Leu Thr Arg Leu Pro Thr Arg Thr Phe Gly Thr
   225                 230                 235                 240
   ctg atg agc atg gaa ccg gcg ctg gct gcc gtt tcc ggg atg att ttc      768
   Leu Met Ser Met Glu Pro Ala Leu Ala Ala Val Ser Gly Met Ile Phe
                   245                 250                 255
   ctc gga gaa aca ctg aca ccc ata cag cta ctg gcg ctc ggc gct atc      816
   Leu Gly Glu Thr Leu Thr Pro Ile Gln Leu Leu Ala Leu Gly Ala Ile
               260                 265                 270
   atc gcc gct tca atg ggg tct acg ctg aca gta cgc aaa gag agc aaa      864
   Ile Ala Ala Ser Met Gly Ser Thr Leu Thr Val Arg Lys Glu Ser Lys
           275                 280                 285
   ata aaa gaa tta gac att aat taa                                      888
   Ile Lys Glu Leu Asp Ile Asn
       290                 295
<210> 12
   <211> 295
   <212> PRT
   <213> Escherichia coli
<400> 12
   Met Pro Gly Ser Leu Arg Lys Met Pro Val Trp Leu Pro Ile Val Ile
   1               5                   10                  15
   Leu Leu Val Ala Met Ala Ser Ile Gln Gly Gly Ala Ser Leu Ala Lys
               20                  25                  30
   Ser Leu Phe Pro Leu Val Gly Ala Pro Gly Val Thr Ala Leu Arg Leu
           35                  40                  45
   Ala Leu Gly Thr Leu Ile Leu Ile Ala Phe Phe Lys Pro Trp Arg Leu
       50                  55                  60
   Arg Phe Ala Lys Glu Gln Arg Leu Pro Leu Leu Phe Tyr Gly Val Ser
   65                  70                  75                  80
   Leu Gly Gly Met Asn Tyr Leu Phe Tyr Leu Ser Ile Gln Thr Val Pro
                   85                  90                  95
   Leu Gly Ile Ala Val Ala Leu Glu Phe Thr Gly Pro Leu Ala Val Ala
               100                 105                 110
   Leu Phe Ser Ser Arg Arg Pro Val Asp Phe Val Trp Val Val Leu Ala
           115                 120                 125
   Val Leu Gly Leu Trp Phe Leu Leu Pro Leu Gly Gln Asp Val Ser His
       130                 135                 140
   Val Asp Leu Thr Gly Cys Ala Leu Ala Leu Gly Ala Gly Ala Cys Trp
   145                 150                 155                 160
   Ala Ile Tyr Ile Leu Ser Gly Gln Arg Ala Gly Ala Glu His Gly Pro
                   165                 170                 175
   Ala Thr Val Ala Ile Gly Ser Leu Ile Ala Ala Leu Ile Phe Val Pro
               180                 185                 190
   Ile Gly Ala Leu Gln Ala Gly Glu Ala Leu Trp His Trp Ser Val Ile
           195                 200                 205
   Pro Leu Gly Leu Ala Val Ala Ile Leu Ser Thr Ala Leu Pro Tyr Ser
   210 215 220
   Leu Glu Met Ile Ala Leu Thr Arg Leu Pro Thr Arg Thr Phe Gly Thr
   225 230 235 240
   Leu Met Ser Met Glu Pro Ala Leu Ala Ala Val Ser Gly Met Ile Phe
   245 250 255
   Leu Gly Glu Thr Leu Thr Pro Ile Gln Leu Leu Ala Leu Gly Ala Ile
   260 265 270
   Ile Ala Ala Ser Met Gly Ser Thr Leu Thr Val Arg Lys Glu Ser Lys
   275 280 285
   Ile Lys Glu Leu Asp Ile Asn
   290 295
<210> 13
   <211> 1026
   <212> DNA
   <213> Escherichia coli
<220>
   <221> CDS
   <222> (1).. (1023)
<400> 13
   atg aaa gcg tta tcc aaa ctg aaa gcg gaa gag ggc atc tgg atg acc 48
   Met Lys Ala Leu Ser Lys Leu Lys Ala Glu Glu Gly Ile Trp Met Thr
   1 5 10 15
   gac gtt cct gta ccg gaa ctc ggg cat aac gat ctg ctg att aaa atc 96
   Asp Val Pro Val Pro Glu Leu Gly His Asn Asp Leu Leu Ile Lys Ile
   20 25 30
   cgt aaa aca gcc atc tgc ggg act gac gtt cac atc tat aac tgg gat 144
   Arg Lys Thr Ala Ile Cys Gly Thr Asp Val His Ile Tyr Asn Trp Asp
   35 40 45
   gag tgg tcg caa aaa acc atc ccg gtg ccg atg gtc gtg ggc cat gaa 192
   Glu Trp Ser Gln Lys Thr Ile Pro Val Pro Met Val Val Gly His Glu
   50 55 60
   tat gtc ggt gaa gtg gta ggt att ggt cag gaa gtg aaa ggc ttc aag 240
   Tyr Val Gly Glu Val Val Gly Ile Gly Gln Glu Val Lys Gly Phe Lys
   65 70 75 80
   atc ggc gat cgc gtt tct ggc gaa ggc cat atc acc tgt ggt cat tgc 288
   Ile Gly Asp Arg Val Ser Gly Glu Gly His Ile Thr Cys Gly His Cys
   85 90 95
   cgc aac tgt cgt ggt ggt cgt acc cat ttg tgc cgc aac acg ata ggc 336
   Arg Asn Cys Arg Gly Gly Arg Thr His Leu Cys Arg Asn Thr Ile Gly
   100 105 110
   gtt ggt gtt aat cgc ccg ggc tgc ttt gcc gaa tat ctg gtg atc ccg 384
   Val Gly Val Asn Arg Pro Gly Cys Phe Ala Glu Tyr Leu Val Ile Pro
   115 120 125
   gca ttc aac gcc ttc aaa atc ccc gac aat att tcc gat gac tta gcc 432
   Ala Phe Asn Ala Phe Lys Ile Pro Asp Asn Ile Ser Asp Asp Leu Ala
   130 135 140
   gca att ttt gat ccc ttc ggt aac gcc gtg cat acc gcg ctg tcg ttt 480
   Ala Ile Phe Asp Pro Phe Gly Asn Ala Val His Thr Ala Leu Ser Phe
   145 150 155 160
   gat ctg gtg ggc gaa gat gtg ctg gtt tct ggt gca ggc ccg att ggt 528
   Asp Leu Val Gly Glu Asp Val Leu Val Ser Gly Ala Gly Pro Ile Gly
   165 170 175
   att atg gca gcg gcg gtg gcg aaa cac gtt ggt gca cgc aat gtg gtg 576
   Ile Met Ala Ala Ala Val Ala Lys His Val Gly Ala Arg Asn Val Val
   180 185 190
   atc act gat gtt aac gaa tac cgc ctt gag ctg gcg cgt aaa atg ggt 624
   Ile Thr Asp Val Asn Glu Tyr Arg Leu Glu Leu Ala Arg Lys Met Gly
   195 200 205
   atc acc cgt gcg gtt aac gtc gcc aaa gaa aat ctc aat gac gtg atg 672
   Ile Thr Arg Ala Val Asn Val Ala Lys Glu Asn Leu Asn Asp Val Met
   210 215 220
   gcg gag tta ggc atg acc gaa ggt ttt gat gtc ggt ctg gaa atg tcc 720
   Ala Glu Leu Gly Met Thr Glu Gly Phe Asp Val Gly Leu Glu Met Ser
   225 230 235 240
   ggt gcg ccg cca gcg ttt cgt acc atg ctt gac acc atg aat cac ggc 768
   Gly Ala Pro Pro Ala Phe Arg Thr Met Leu Asp Thr Met Asn His Gly
   245 250 255
   ggc cgt att gcg atg ctg ggt att ccg ccg tct gat atg tct atc gac 816
   Gly Arg Ile Ala Met Leu Gly Ile Pro Pro Ser Asp Met Ser Ile Asp
   260 265 270
   tgg acc aaa gtg atc ttt aaa ggc ttg ttc att aaa ggt att tac ggt 864
   Trp Thr Lys Val Ile Phe Lys Gly Leu Phe Ile Lys Gly Ile Tyr Gly
   275 280 285
   cgt gag atg ttt gaa acc tgg tac aag atg gcg gcg ctg att cag tct 912
   Arg Glu Met Phe Glu Thr Trp Tyr Lys Met Ala Ala Leu Ile Gln Ser
   290 295 300
   ggc ctc gat ctt tcg ccg atc att acc cat cgt ttc tct atc gat gat 960
   Gly Leu Asp Leu Ser Pro Ile Ile Thr His Arg Phe Ser Ile Asp Asp
   305 310 315 320
   ttc cag aag ggc ttt gac gct atg cgt tcg ggc cag tcc ggg aaa gtt 1008
   Phe Gln Lys Gly Phe Asp Ala Met Arg Ser Gly Gln Ser Gly Lys Val
   325 330 335
   att ctg agc tgg gat taa 1026
   Ile Leu Ser Trp Asp
   340
<210> 14
   <211> 341
   <212> PRT
   <213> Escherichia coli
<400> 14
   Met Lys Ala Leu Ser Lys Leu Lys Ala Glu Glu Gly Ile Trp Met Thr
   1 5 10 15
   Asp Val Pro Val Pro Glu Leu Gly His Asn Asp Leu Leu Ile Lys Ile
   20 25 30
   Arg Lys Thr Ala Ile Cys Gly Thr Asp Val His Ile Tyr Asn Trp Asp
   35 40 45
   Glu Trp Ser Gln Lys Thr Ile Pro Val Pro Met Val Val Gly His Glu
   50 55 60
   Tyr Val Gly Glu Val Val Gly Ile Gly Gln Glu Val Lys Gly Phe Lys
   65 70 75 80
   Ile Gly Asp Arg Val Ser Gly Glu Gly His Ile Thr Cys Gly His Cys
   85 90 95
   Arg Asn Cys Arg Gly Gly Arg Thr His Leu Cys Arg Asn Thr Ile Gly
   100 105 110
   Val Gly Val Asn Arg Pro Gly Cys Phe Ala Glu Tyr Leu Val Ile Pro
   115 120 125
   Ala Phe Asn Ala Phe Lys Ile Pro Asp Asn Ile Ser Asp Asp Leu Ala
   130 135 140
   Ala Ile Phe Asp Pro Phe Gly Asn Ala Val His Thr Ala Leu Ser Phe
   145 150 155 160
   Asp Leu Val Gly Glu Asp Val Leu Val Ser Gly Ala Gly Pro Ile Gly
   165 170 175
   Ile Met Ala Ala Ala Val Ala Lys His Val Gly Ala Arg Asn Val Val
   180 185 190
   Ile Thr Asp Val Asn Glu Tyr Arg Leu Glu Leu Ala Arg Lys Met Gly
   195 200 205
   Ile Thr Arg Ala Val Asn Val Ala Lys Glu Asn Leu Asn Asp Val Met
   210 215 220
   Ala Glu Leu Gly Met Thr Glu Gly Phe Asp Val Gly Leu Glu Met Ser
   225 230 235 240
   Gly Ala Pro Pro Ala Phe Arg Thr Met Leu Asp Thr Met Asn His Gly
   245 250 255
   Gly Arg Ile Ala Met Leu Gly Ile Pro Pro Ser Asp Met Ser Ile Asp
   260 265 270
   Trp Thr Lys Val Ile Phe Lys Gly Leu Phe Ile Lys Gly Ile Tyr Gly
   275 280 285
   Arg Glu Met Phe Glu Thr Trp Tyr Lys Met Ala Ala Leu Ile Gln Ser
   290 295 300
   Gly Leu Asp Leu Ser Pro Ile Ile Thr His Arg Phe Ser Ile Asp Asp
   305                 310                 315                 320
   Phe Gln Lys Gly Phe Asp Ala Met Arg Ser Gly Gln Ser Gly Lys Val
                   325                 330                 335
   Ile Leu Ser Trp Asp
               340

## Description

### Technical Field

The present invention relates to the microbiological industry, and specifically to a method for producing an L-amino acid using a bacterium of the E*nterobacteriaceae* family, which has been modified to attenuate expression of the *yahN* gene.

### Background Art

Conventionally, L-amino acids are industrially produced by fermentation methods utilizing strains of microorganisms obtained from natural sources, or mutants thereof. Typically, the microorganisms are modified to enhance production yields of L-amino acids.

Some proteins of E. *coli* were described which, when over-expressed, conferred cells resistance to threonine and homoserine (Livshits V.A., et al., Res Microbiol., 154(2), 123, 123-35 (2003); Zakataeva N.P., et al.. FEBS Lett., 452. 228-232 (1999)). These genes were shown to encode proteins involved in amino acid efflux. Genome sequence analysis of *E*. *coli* and other prokaryotes revealed that RhtB and RhtC proteins belong to a novel RhtB family of membrane proteins (Aleshin V.V., et al., Trends Biochem Sci., 24(4)., 133-5(1999)). This family also included *E*. *coli* proteins encoded by the *yfiK*, *yahN*, *yeaS*, and *yggA* genes. The amplification of *yahN* DNA fragments resulted in increased proline and glutamic acid accumulation and cell resistance to L-amino acids from glutamate family (EP 101610A2). WO2006057341 discloses an E. coli strain lacking the yahn gene.

But currently, there have been no reports of attenuating expression of the *yahN* gene for producing of L-amino acids of aspartate family.

### Disclosure of the Invention

Objects of present invention are to enhance the productivity of L-amino acid producing strains and to provide a method for producing an L-amino acid using these strains.

The present invention provides a method using a bacterium of the *Enterobacteriaceae* family having an increased ability to produce L-threonine,

wherein the bacterium has been modified to attenuate expression of the *yahN* gene, such that it is unable to synthesize the YahN protein.

It is a further aspect of the present invention to provide the method as described above, wherein the expression of the *yahN* gene is attenuated by inactivation of the *yahN* gene.

It is a further aspect of the present invention to provide the method as described above, wherein the bacterium belongs to the genus *Escherichia*.

It is a further aspect of the present invention to provide the method described above, wherein the bacterium belongs to Escherichia coli.

The present invention is described in detail below.

### Brief Description of Drawings

Figure 1 shows the relative positions of primers yahN L and yahN R on plasmid pACYC177, which is used for amplification of the *cat* gene.

Figure 2 shows the construction of the chromosomal DNA fragment comprising the inactivated *yahN* gene.

### Detailed Description of the Preferred Embodiments

### 1. Bacterium of the present invention

The bacterium for use in a method of the present invention is an L-amino acid producing bacterium of the *Enterobacteriaceae* family, wherein the bacterium has been modified to attenuate expression of the *yahN* gene.

In the present invention, "L-amino acid producing bacterium" means a bacterium, which is able to produce and excrete L-amino acid into a medium, when the bacterium is cultured in the medium.

The phrase "L-amino acid-producing bacterium" as used herein also means a bacterium which is able to produce and cause accumulation of an L-amino acid in a culture medium in an amount larger than a wild-type or parental strain of the bacterium, for example, *E*. *coli,* such as *E*. *coli* K-12, and preferably means that the microorganism is able to cause accumulation in a medium of an amount not less than 0.5 g/L, more preferably not less than 1.0 g/L of the target L-amino acid. The term "L-amino acid of aspartate family" includes L-asparagine, L-threonine, L-lysine, L-methionine, L-alanine and L-homoserine.

The *Enterobacteriaceae* family includes bacteria belonging to the genera *Escherichia*, *Enterobacter*, *Erwinia*, *Klebsiella*, *Pantoea*, *Photorhabdus*, *Providencia, Salmonella*, *Serratia*, *Shigella*, *Morganella Yersinia*, etc.. Specifically, those classified into the *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used. A bacterium belonging to the genus *Escherichia* or *Pantoea* is preferred.

The phrase "a bacterium belonging to the genus *Escherichia*" means that the bacterium is classified in the genus *Escherichia* according to the classification known to a person skilled in the art of microbiology. Examples of a bacterium belonging to the genus *Escherichia* as used in the present invention include, but are not limited to, *Escherichia coli* (*E. coli*).

The bacterium belonging to the genus *Escherichia* that can be used in the present invention is not particularly limited, however for example, bacteria described by Neidhardt, F.C. et al. (Escherichia coli and Salmonella typhimurium, American Society for Microbiology, Washington D.C., 1208, Table 1) are encompassed by the bacterium of the present invention.

The phrase "bacterium has been modified to attenuate expression of the *yahN* gene" means that the bacterium has been modified in such a way that a modified bacterium is unable to synthesize the YahN protein.

The phrase "inactivation of the *yahN* gene" means that the modified gene encodes a completely non-functional protein. It is also possible that the modified DNA region is unable to naturally express the gene due to the deletion of a part of the gene, the shifting of the reading frame of the gene, the introduction of missense/nonsense mutation(s), or the modification of an adjacent region of the gene, including sequences controlling gene expression, such as a promoter, enhancer, attenuator, ribosome-binding site, etc.

The presence or absence of the *yahN* gene in the chromosome of a bacterium can be detected by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the protein encoded by the *yahN* gene can be measured by well-known methods, including SDS-PAGE followed by immunoblotting assay (Western blotting analysis), and the like.

The *yahN* gene (synonyms: *b0328*) encodes the YahN protein (synonyms: putative cytochrome subunit of dehydrogenase, B0328, YahN). The *yahN* gene (nucleotides complementary to nucleotides 344,890 to 345,561 in the GenBank accession number NC_000913.2; gi:49175990; SEQ ID NO: 1) is located between the *yahM* and the *yahO* genes on the chromosome of *E*. *coli* strain K-12. The nucleotide sequence of the *yahN* gene and the amino acid sequence of YahN protein encoded by the *yahN* gene are shown in SEQ ID NO: 1 and SEQ ID NO:2, respectively.

Since there may be some differences in DNA sequences between the genera or strains of the *Enterobacteriaceae* family, the gene to be attenuated or inactivated on the chromosome is not limited to the gene shown in SEQ ID No:1, but may include genes homologous to SEQ ID No: 1 which encode a variant protein of the YahN protein(s). The phrase "variant protein" as used in the present invention means a protein which has changes in the sequence, whether they are deletions, insertions, additions, or substitutions of amino acids, but still maintains the activity of the product as the YahN protein. The number of changes in the variant protein depends on the position or the type of amino acid residues in the three dimensional structure of the protein. It may be 1 to 30, preferably 1 to 15, and more preferably 1 to 5 in SEQ ID NO: 2. These changes in the variants can occur in regions of the protein which are not critical for the function of the protein. This is because some amino acids have high homology to one another so the three dimensional structure or activity is not affected by such a change. Therefore, the protein variant encoded by the *yahN* gene may have a homology of not less than 80 %, preferably not less than 90%, and most preferably not less than 95 %, with respect to the entire amino acid sequence shown in SEQ ID NO: 2, as long as the ability of the YahN protein prior to inactivation is maintained.

Homology between two amino acid sequences can be determined using the well-known methods, for example, the computer program BLAST 2.0, which calculates three parameters: score, identity and similarity.

Moreover, the *yahN* gene may be a variant which hybridizes with the nucleotide sequence shown in SEQ ID NO: 1, or a probe which can be prepared from the nucleotide sequence under stringent conditions, provided that it encodes a functional YahN protein prior to inactivation. "Stringent conditions" include those under which a specific hybrid, for example, a hybrid having homology of not less than 60%, preferably not less than 70%, more preferably not less than 80%, still more preferably not less than 90%, and most preferably not less than 95%, is formed and a non-specific hybrid, for example, a hybrid having homology lower than the above, is not formed. For example, stringent conditions are exemplified by washing one time, preferably two or three times at a salt concentration corresponding to 1 × SSC, 0.1% SDS, preferably 0.1 × SSC, 0.1% SDS at 60°C. The length of the probe may be suitably selected depending on the hybridization conditions, and is usually 100 bp to 1 kbp.

Expression of the *yahN* gene can be attenuated by introducing a mutation into the gene on the chromosome so that intracellular activity of the protein encoded by the gene is decreased as compared with an unmodified strain. Such a mutation on the gene can be replacement of one base or more to cause an amino acid substitution in the protein encoded by the gene (missense mutation), introduction of a stop codon (nonsense mutation), deletion of one or two bases to cause a frame shift, insertion of a drug-resistance gene, or deletion of a part of the gene or the entire gene (Qiu, Z. and Goodman, M.F., J. Biol. Chem., 272, 8611-8617 (1997); Kwon, D. H. et al, J. Antimicrob. Chemother., 46, 793-796 (2000)). Expression of the *yahN* gene can also be attenuated by modifying an expression regulating sequence such as the promoter, the Shine-Dalgamo (SD) sequence, etc. (WO95/34672, Carrier, T.A. and Keasling, J.D., Biotechnol Prog 15, 58-64 (1999)).

For example, the following methods may be employed to introduce a mutation by gene recombination. A mutant gene encoding a mutant protein having a decreased activity is prepared, and a bacterium to be modified is transformed with a DNA fragment containing the mutant gene. Then the native gene on the chromosome is replaced with the mutant gene by homologous recombination, and the resulting strain is selected. Such gene replacement using homologous recombination can be conducted by the method employing a linear DNA, which is known as "Red-driven integration" (Datsenko, K.A. and Wanner, B.L., Proc. Natl. Acad. Sci. USA, 97, 12, p 6640-6645 (2000)), or by employing a plasmid containing a temperature-sensitive replication control region (U.S. Patent 6,303,383 or JP 05-007491 A). Furthermore, the incorporation of a site-specific mutation by gene substitution using homologous recombination such as set forth above can also be conducted with a plasmid lacking the ability to replicate in the host.

Expression of the gene can also be attenuated by insertion of a transposon or an IS factor into the coding region of the gene (U.S. Patent No. 5,175,107), or by conventional methods, such as mutagenesis treatment using UV irradiation or nitrosoguanidine (N-methyl-N'-nitro-N-nitrosoguanidine).

Inactivation of the gene can also be performed by conventional methods, such as a mutagenesis using UV irradiation or nitrosoguanidine (N-methyl-N'-nitro-N-nitrosoguanidine), site-directed mutagenesis, gene disruption using homologous recombination, or/and insertion-deletion mutagenesis (Yu, D. et al., Proc. Natl. Acad. Sci. USA, 2000, 97:12: 5978-83 and Datsenko, K.A. and Wanner, B.L., Proc. Natl. Acad. Sci. USA, 2000, 97:12: 6640-45), also called "Red-driven integration".

Activity of the YahN protein can be detected by, for example, overexpressing a *yahN* gene in a bacterium, and confirming whether L-proline or L-glutamic acid production by the bacterium or resistance of the bacterium to L-amino acids from glutamate family is increased (EP 1016710A2). So, the reduced or absent activity of the YahN protein in the bacterium for use in a method according the present invention can be determined by comparing such an activity to the parent unmodified bacterium.

Methods for preparation of plasmid DNA, digestion and ligation of DNA, transformation, selection of an oligonucleotide as a primer, and the like may be ordinary methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

### L- amino acid producing bacteria

As a bacterium for use in a method of the present invention, which is modified to attenuate expression of the *yahN* gene, bacteria, which are able to produce L-threonine may be used.

The bacterium for use in a method of the present invention can be obtained by inactivating the *yahN* gene in a bacterium which inherently has the ability to produce L-threonine. Alternatively, the bacterium of present invention can be obtained by imparting the ability to produce L-threonine to a bacterium already having the *yahN* gene inactivated.

### L-threonine-producing bacteria

Examples of parent strains for deriving the L-threonine-producing bacteria for use in a method of the present invention include, but are not limited to, L-threonine-producing bacteria belonging to the genus *Escherichia,* such as *E. coli* strain TDH-6/pVIC40 (VKPM B-3996) (US Patent 5,175,107, US Patent 5,705,371), *E. coli* strain NRRL-21593 (US Patent 5,939,307), *E. coli* strain FERM BP-3756 (US Patent 5,474,918), *E. coli* strains FERM BP-3519 and FERM BP-3520 (US Patent 5,376,538), *E. coli* strain MG442 (Gusyatiner et al., Genetika (in Russian), 14, 947-956 (1978)), *E. coli* strains VL643 and VL2055 (EP 1149911 A), and the like.

The strain TDH-6 is deficient in the *thrC* gene, as well as being sucrose-assimilative, and the *ilvA* gene has a leaky mutation. This strain also has a mutation in the *rhtA* gene, which imparts resistance to high concentrations of threonine or homoserine. The strain VKPM B-3996 contains the plasmid pVIC40 which was obtained by inserting a *thr A*BC* operon which includes a mutant *thrA* gene into a RSF1010-derived vector. This mutant *thrA gene* encodes aspartokinase homoserine dehydrogenase I which is substantially desensitized to feedback inhibition by threonine. The strain B-3996 was deposited on November 19, 1987 in the All-Union Scientific Center of Antibiotics (Nagatinskaya Street 3-A, 117105 Moscow, Russian Federation) under the accession number RIA 1867. The strain was also deposited in the Russian National Collection of Industrial Microorganisms (VKPM) (1 Dorozhny proezd 1, Moscow 117545, Russian Federation) on April 7, 1987 under the accession number VKPM B-3996.

Preferably, the bacterium for use in a method of the present invention is further modified to enhance expression of one or more of the following genes:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase);

The *thrA* gene which encodes aspartokinase homoserine dehydrogenase I of *Escherichia coli* has been elucidated (nucleotide numbers 337 to 2799 in the sequence of GenBank accession NC_000913.2, gi: 49175990). The *thrA* gene is located between *thrL* and *thrB* genes on the chromosome *of E. coli* K-12. The *thrB* gene which encodes homoserine kinase of *Escherichia coli* has been elucidated (nucleotide numbers 2801 to 3733 in the sequence of GenBank accession NC_000913.2, gi: 49175990). The *thrB* gene is located between *thrA* and *thrC* genes on the chromosome *of E. coli* K-12. The *thrC* gene which encodes threonine synthase of *Escherichia coli* has been elucidated (nucleotide numbers 3734 to 5020 in the sequence of GenBank accession NC_000913.2, gi: 49175990). The *thrC* gene is located between *thrB* gene and *yaaX* opened reading frame on the chromosome *of E. coli* K-12. All three genes function as a single threonine operon.

A mutant *thrA* gene which codes for aspartokinase homoserine dehydrogenase I resistant to feedback inhibition by threonine, as well as, the *thrB* and *thrC* genes can be obtained as one operon from the well-known plasmid pVIC40 which is present in the threonine producing E. *coli* strain VKPM B-3996. Plasmid pVIC40 is described in detail in US Patent 5,705,371.

The *rhtA* gene exists at 18 min on *E. coli* chromosome close to the *glnHPQ* operon that encodes components of the glutamine transport system, and the *rhtA* gene is identical to ORF1 (*ybiF* gene, numbers 764 to 1651 in the GenBank accession number AAA218541, gi:440181 : SEQ ID NO: 11), located between the *pexB* and *ompX* genes. The unit expressing a protein encoded by the ORF1 has been designated *rhtA* (rht: resistance to homoserine and threonine) gene. Also, it was found that the *rhtA23* mutation is an A-for-G substitution at position -1 with respect to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457, EP 1013765 A). The *rhtA* gene may be a homologous gene having not less than 80% homology, preferably 90% homology, more preferably 95% homology to SEQ ID NO: 11 as long as it enhances L-threonine producing ability of a bacterium when its expression is enhanced in the bacterium.

The *asd* gene of *E.coli* has already been elucidated (nucleotide numbers 3572511 to 3571408 in the sequence of GenBank accession NC_000913.1, gi:16131307), and can be obtained by PCR (polymerase chain reaction; refer to White, T.J. et al., Trends Genet., 5, 185 (1989)) utilizing primers based on the nucleotide sequence of the gene. The *asd* genes of other microorganisms can be obtained in a similar manner.

Also, the *aspC* gene of *E. coli* has already been elucidated (nucleotide numbers 983742 to 984932 in the sequence of GenBank accession NC_000913.1, gi:1618895), and can be obtained by PCR. The *aspC* genes of other microorganisms can be obtained in a similar manner.

Another example of a parent strain for deriving the L-threonine-producing bacteria for use in a method of the present invention includes a bacterium which is deficient in enzymatic activity of L-threonine decomposing enzyme. An example of the L-threonine decomposing enzyme includes threonine dehydrogenase (tdh). The nucleotide sequence of a tdh gene *of E. coli* has already been elucidated (SEQ ID NO: 13). The *tdh* gene may be a homologous gene having not less than 80% homology, preferably 90% homology, more preferably 95% homology to SEQ ID NO: 13 as long as it encodes a protein having threonine dehydrogenase activity.

### 2. Method of the present invention

The method of the present invention is a method for producing L-threonine acid by cultivating the bacterium as described herein in a culture medium to produce and excrete the L-amino acid into the medium, and collecting the L-amino acid from the medium.

In the present invention, the cultivation, collection, and purification of L-amino acid from the medium and the like may be performed in a manner similar to conventional fermentation methods wherein an amino acid is produced using a bacterium.

The medium used for culture may be either synthetic or natural, so long as it includes a carbon source, a nitrogen source, minerals and, if necessary, appropriate amounts of nutrients which the bacterium requires for growth. The carbon source may include various carbohydrates such as glucose and sucrose, and various organic acids. Depending on the mode of assimilation of the chosen microorganism, alcohol, including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used. As vitamins, thiamine, yeast extract, and the like, can be used.

The cultivation is preferably performed under aerobic conditions, such as a shaking culture, and a stirring culture with aeration, at a temperature of 20 to 40 °C, preferably 30 to 38 °C. The pH of the culture is usually between 5 and 9, preferably between 6.5 and 7.2. The pH of the culture can be adjusted with ammonia, calcium carbonate, various acids, various bases, and buffers. Usually. 1 to 5-day cultivation leads to accumulation of the target L-amino acid in the liquid medium.

After cultivation, solids such as cells can be removed from the liquid medium by centrifugation or membrane filtration, and then the L-amino acid can be collected and purified by ion-exchange, concentration, and/or crystallization methods.

### Examples

The present invention will be more concretely explained below with reference to the following non-limiting Examples.

### Example 1. Construction of a strain with an inactivated yahN gene.

### 1. Deletion of the yahN gene.

A strain in which the *yahN* gene has been deleted was constructed by the method initially developed by Datsenko, K.A. and Wanner, B.L. (Proc. Natl. Acad. Sci. USA, 2000, 97(12), 6640-6645) called "Red-driven integration". According to this procedure, the PCR primers yahN L (SEQ ID NO: 3) and yahN R (SEQ ID NO: 4), which are homologous to both the region adjacent to the *yahN* gene and the gene conferring antibiotic resistance in the template plasmid, were constructed. The plasmid pACYC177 (GenBank/EMBL accession number X06402) is used as a template in the PCR reaction. Conditions for PCR were as follows: initial DNA denaturation for 5 min at 95 °C, followed by 25 cycles of denaturation at 95 °C for 30 sec, annealing at 54 °C for 30 sec, elongation at 72 °C for 40 sec; and the final elongation for 5 min at 72 °C.

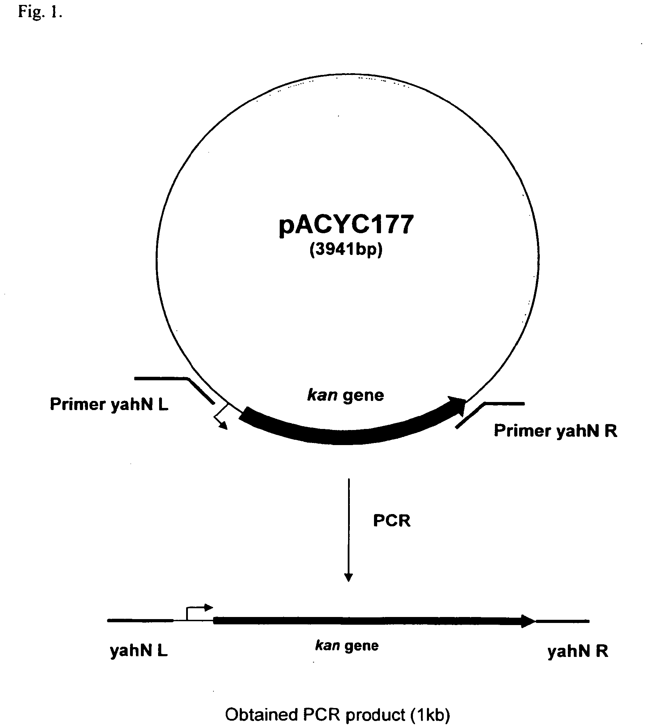

A 1 kb PCR product (Fig. 1) was obtained and purified in agarose gel and was used for electroporation of the *E*. *coli* strain MG1655 (ATCC 700926), which contains the plasmid pKD46 having a temperature-sensitive replication control region. The strain MG1655 can be obtained from American Type Culture Collection. (P.O. Box 1549 Manassas, VA 20108, United States of America).

The recombinant plasmid pKD46 (Datsenko, K.A., Wanner, B.L., Proc. Natl. Acad. Sci. USA, 2000, 97, 6640-6645) with the thermosensitive replicon was used as the donor of the phage λ-derived genes responsible for the Red-mediated recombination system. The plasmid pKD46 includes a 2,154 nucleotide (31088-33241) DNA fragment of phage λ (GenBank accession No. J02459), and contains genes of the λ Red homologous recombination system (y, β, exo genes) under the control of the arabinose-inducible P_{araB} promoter. The plasmid pKD46 is necessary for integration of the PCR product into the chromosome of strain MG1655. *E. coli* BW25113 containing the recombinant plasmid pKD46 can be obtained from the E. *coli* Genetic Stock Center, Yale University, New Haven, USA, the accession number of which is CGSC7630.

The obtained DNA fragment was used for electroporation and Red-mediated integration into the bacterial chromosome of the E. *coli* MG1655/pKD46.

Electrocompetent cells were prepared as follows: MG1655/pKD46 cells were grown overnight at 30 °C in the liquid LB-medium with addition of ampicillin (100 µg/ml), then diluted 1:100 by the SOB-medium (Yeast extract, 5 g/l; NaCl, 0.5 g/l; Tryptone, 20 g/l; KCl, 2.5 mM; MgCl₂, 10 mM) with addition of ampicillin (100 µg/ml) and L-arabinose (10 mM) (arabinose is used for inducing the plasmid encoding genes of Red system) and grown at 30 °C to reach the optical density of the bacterial culture OD₆₀₀=0.4-0.7. The grown cells from 10 ml of the bacterial culture were washed 3 times by the ice-cold de-ionized water, followed by suspending in 100 µl of the water.

Electroporation was performed using 70 µl of cells and 10 µl of PCR product (≈100 ng) dissolved in the de-ionized water. Shocked cells were added to 1-ml of SOC medium (Sambrook et al, "Molecular Cloning A Laboratory Manual, Second Edition", Cold Spring Harbor Laboratory Press (1989)) at 37 °C for 2 hours, and after that plated onto L-agar containing 20 µg/ml of kanamycin to select kanamycin-resistant (Km^{R}) recombinants. Colonies grown within 24 h were tested for the presence of Km^{R} marker instead of native *yahN* gene. A strain introduced with the Km^{R} marker gene was selected and cured from the thermosensitive plasmid pKD46 by culturing at 37 °C, and the resulting strain was named as E. *coli* MG1655ΔyahN.

### 2. Verification of the yahN gene deletion by PCR.

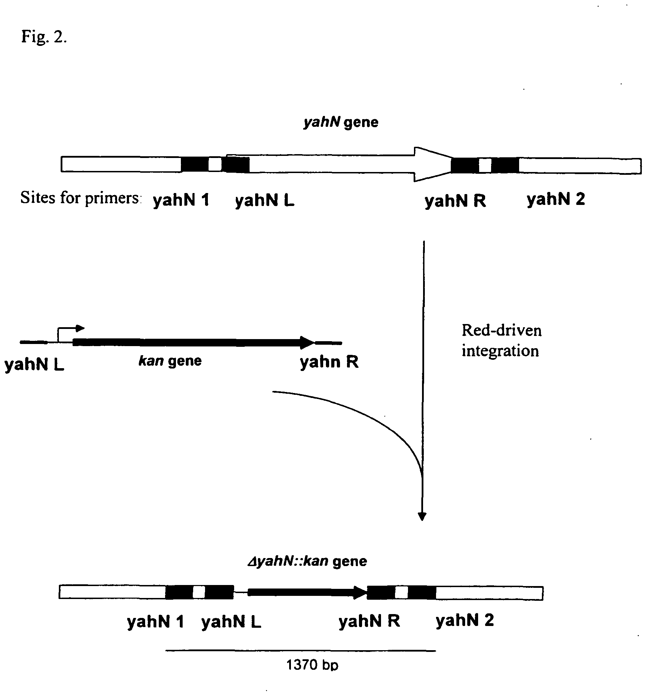

The mutants, which have the *yahN* gene deleted, marked with Km resistance gene, were verified by PCR. Locus-specific primers yahN 1 (SEQ ID NO: 5) and yahN 2 (SEQ ID NO: 6) were used in PCR for the verification. For this purpose, a freshly isolated colony was suspended in 20µl water and then 1µl of obtained suspension was used for PCR. The temperature profile follows: initial DNA denaturation for 5 min at 95 °C; then 30 cycles of denaturation at 95 °C for 30 sec, annealing at 55 °C for 30 sec and elongation at 72 °C for 1 min; the final elongation for 5 min at 72 °C. The PCR product obtained in the reaction with the cells of parental yahN ⁺ strain MG 1655 as a template, was 1060 bp in length. The PCR product obtained in the reaction with the cells of mutant MG1655 ΔyahN::kan strain as the template was 1370 nucleotides in length (Fig.2).

### Example 2. Production of L-threonine by E. coli strain MG1655Δtdh::rhtA,ΔyahN (pVIC40).

To test the effect of inactivation of the *yahN* gene on L-threonine production, the *E. coli* strain MG1655Δtdh::rhtA, ΔyahN (pVIC40) was constructed as follows.

At first, the E. *coli* strain MG1655 Δtdh::rhtA* (pVIC40) was prepared.

The L-threonine producing *E*. *coli* strain MG1655 Δtdh, rhtA* (pVIC40) was constructed by inactivation of the native *tdh* gene (SEQ ID NO: 13) in E. *coli* MG1655 using the *cat* gene followed by introduction of an rhtA23 mutation which confers resistance to high concentrations of threonine (>40 mg/ml) and homoserine (>5 mg/ml). Then, the resulting strain was transformed with plasmid pVIC40 from *E*. *coli* VKPM B-3996. Plasmid pVIC40 is described in detail in the US patent 5,705, 371.

To substitute the native *tdh* gene, a DNA fragment carrying the chloramphenicol resistance marker (Cm^{R}) encoded by the *cat* gene was integrated into the chromosome of E. *coli* MG1655 (ATCC 700926) in place of the native gene by the method described by Datsenko K.A. and Wanner B.L. (Proc.Natl.Acad.Sci.USA, 2000, 97, 6640-6645) which is also called "Red-mediated integration " and/or "Red-driven integration" as described in the Example 1.

A DNA fragment containing a Cm^{R} marker encoded by the *cat* gene was obtained by PCR using the commercially available plasmid pACYC184 (GenBank/EMBL accession number X06403, "Fermentas", Lithuania) as the template, and primers tdhL (SEQ ID NO: 7) and tdhR (SEQ ID NO: 8). Primer P1 contains 35 nucleotides homologous to the 5'-region of the *tdh* gene introduced into the primer for further integration into the bacterial chromosome. Primer P2 contains 32 nucleotides homologous to the 3'-region of the *tdh* gene introduced into the primer for further integration into the bacterial chromosome.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added in the reaction mixture as a template DNA for the PCR amplification. The temperature profile was the following: initial DNA denaturation for min at 95 °C, followed by 25 cycles of denaturation at 95 °C for 30 sec, annealing at 55 °C for 30 sec, elongation at 72 °C for 40 sec; and the final elongation for 5 min at 72 °C. Then, the amplified DNA fragment was purified by agarose gel-electrophoresis, extracted using "GenElute Spin Columns" (Sigma, USA), and precipitated by ethanol.

The obtained DNA fragment was used for electroporation and Red-mediated integration into the bacterial chromosome of the E. *coli* MG1655/pKD46.

MG1655/pKD46 cells were grown overnight at 30 °C in the liquid LB-medium with addition of ampicillin (100 µg/ml), then diluted 1:100 by the SOB-medium (Yeast extract, 5 g/l; NaCl, 0.5 g/l; Tryptone, 20 g/l; KCl, 2.5 mM; MgCl₂, 10 mM) with addition of ampicillin (100 µg/ml) and L-arabinose (10 mM) (arabinose is used for inducing the plasmid containing the genes of the Red system) and grown at 30 °C to reach the optical density of the bacterial culture OD₆₀₀=0.4-0.7. The grown cells from 10 ml of the bacterial culture were washed 3 times with ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al, "Molecular Cloning A Laboratory Manual, Second Edition", Cold Spring Harbor Laboratory Press (1989)), incubated 2 hours at 37 °C, and then were spread onto L-agar containing 25 µg/ml of chloramphenicol. Colonies grown for 24 h were tested for the presence of Cm^{R} marker instead of the native *tdh* gene by PCR using primers tdh1 (SEQ ID NO: 9) and tdh2 (SEQ ID NO: 10). For this purpose, a freshly isolated colony was suspended in 20µl water and then 1µl of obtained suspension was used for PCR. The temperature profile follows: initial DNA denaturation for 5 min at 95 °C; then 30 cycles of denaturation at 95 °C for 30 sec, annealing at 55 °C for 30 sec and elongation at 72 °C for 30 sec; the final elongation for 5 min at 72 °C. A few Cm^{R} colonies tested contained the desired 1104 bp DNA fragment, confirming the presence of Cm^{R} marker DNA instead of 1242 bp fragment of *tdh* gene. One of the obtained strains was cured from the thermosensitive plasmid pKD46 by culturing at 37 °C and the resulting strain was named as *E. coli* MG1655Δtdh.

Then, the rhtA23 mutation from the strain VL614rhtA23 (Livshits V.A. et al, 2003, Res. Microbiol., 154:123-135) was introduced into obtained strain MG1655 Δtdh resulting in the strain MG1655 Δtdh, rhtA*. The rhtA23 is a mutation which confers resistance to highconcentrations of threonine (>40 mg/ml) and homoserine (>5 mg/ml). For that purpose the strain MG 1655 Δtdh was infected with phage P1ᵥᵢᵣ grown on the donor strain VL614rhtA23. The transductants were selected on M9 minimal medium containing 8 mg/ml homoserine and 0.4% glucose as the sole carbon source.

DNA fragments from the chromosome of the above-described *E*. *coli* MG1655Δ yahN::kan strain were transferred to the threonine-producing *E*. *coli* strain MG1655Δtdh::rhtA by P1 transduction (Miller, J.H. (1972) Experiments in Molecular Genetics, Cold Spring Harbor Lab. Press, Plainview, NY). The strain MG1655Δtdh::rhtA,ΔyahN was obtained.

The strain MG1655Δtdh::rhtA,ΔyahN and a parent strain MG1655Δtdh::rhtA were each transformed with pVIC40 plasmid and cultivated at 37 °C for 18 hours in a nutrient broth and 0.3 ml of each of the obtained cultures was inoculated into 3 ml of fermentation medium having the following composition in a 20x200 mm test tube and cultivated at 37 °C for 24 h with a rotary shaker.

### Fermentation medium composition (g/l):

| | |
|---|---|
| Glucose | 40 |
| (NH₄)₂SO₄ | 16 |
| K₂HPO₄ | 0.7 |
| MgSO₄·7H₂O | 1.0 |
| MnSO₄·5H₂O | 0.01 |
| FeSO₄·7H₂O | 0.01 |
| Thiamine hydrochloride | 0.002 |
| Yeast extract | 2.0 |
| L-isoleucine | 0.01 |
| CaCO₃ | 33 |

MsSO₄·7H₂O and CaCO₃ were each sterilized separately.

After cultivation, the amount of L-threonine which had accumulated in the medium was determined by paper chromatography using the following mobile phase: butanol : acetic acid : water = 4 : 1 : 1 (v/v). A solution (2%) of ninhydrin in acetone was used as a visualizing reagent. A spot containing L-threonine was cut off, L-threonine was eluted in 0.5 % water solution of CdCl₂, and the amount of L-threonine was estimated spectrophotometrically at 540 nm.

The results of 20 independent test tube fermentations are shown in Table 1. As it can be seen from the Table 1, MG1655Δtdh::rhtA,ΔyahN (pVIC40) caused a higher amount of accumulation of L-threonine as compared with MG1655Δtdh::rhtA (pVIC40).

**Table 1**

| Strain | OD₅₄₀ | Amount of L-threonine_{;} g/l |
|---|---|---|
| MG1655Δtdh::rhtA(pVIC40) | 30.4 ± 0.6 | 7.0±0.4 |
| MG) 655Atdh::rhtA,ΔyahN(pV!C40) | 29.4 ± 0.9 | 7.9±0.5 |

### Industrial Applicability

According to the present invention, production of L-threonine of a bacterium of the *Enterobacteriaceae* family can be enhanced.

## Claims

1. A method for producing L-threonine comprising:
- cultivating a bacterium of the *E*nterobacteriaceae family producing L-threonine in a medium to produce and excrete L-threonine into the medium, and
- collecting said L-threonine from the medium,
wherein said bacterium has been modified to attenuate expression of the *yahN* gene, such that it is unable to synthesize the YahN protein.

2. The method according to claim 1, wherein said expression of the *yahN* gene is attenuated by inactivation of the *yahN* gene.

3. The method according to claim 1, wherein said bacterium belongs to the genus *Escherichia.*

4. The method according to claim 1, wherein said bacterium belongs to *Escherichia coli.*

## Patentansprüche

1. Verfahren zur Herstellung von L-Threonin, umfassend:
- Kultivierung eines Bakteriums der Familie Enterobacteriaceae, das L-Threonin herstellt, in einem Medium, um L-Threonin herzustellen und in das Medium auszuscheiden, und
- Gewinnen des L-Threonins aus dem Medium,
wobei das Bakterium modifiziert wurde, um die Expression des yahN-Gens abzuschwächen, so dass es unfähig ist, das YahN-Protein zu synthetisieren.

2. Verfahren gemäß Anspruch 1, wobei die Expression des yahN-Gens durch Inaktivierung des yahN-Gens abgeschwächt ist.

3. Verfahren gemäß Anspruch 1, wobei das Bakterium zur Gattung Escherichia gehört.

4. Verfahren gemäß Anspruch 1, wobei das Bakterium zu Escherichia coli gehört.

## Revendications

1. Procédé pour produire de la L-thréonine comprenant :
- la culture d'une bactérie de la famille *Enterobacteriaceae* produisant de la L-thréonine dans un milieu pour produire et excréter de la L-thréonine dans le milieu, et
- la collecte de ladite L-thréonine à partir du milieu,
dans lequel ladite bactérie a été modifiée pour atténuer l'expression du gène *yahN,* de sorte qu'il est incapable de synthétiser la protéine YahN.

2. Procédé selon la revendication 1, dans lequel ladite expression du gène *yahN* est atténuée par l'inactivation du gène *yahN.*

3. Procédé selon la revendication 1, dans lequel ladite bactérie appartient au genre *Escherichia.*

4. Procédé selon la revendication 1, dans lequel ladite bactérie appartient à *Escherichia coli.*
